# EUROPEAN PATENT APPLICATION

(11) **EP 3 912 976 A1**
(43) Date of publication of application: **24.11.2021**
(21) Application number: 20741763.5
(22) Date of filing: 17.01.2020
(51) Int. Cl.: C07D 401/12, A61K 31/505, A61K 31/675, A61P 35/00

(54) **SALT OF EGFR INHIBITOR, CRYSTAL FORM, AND PREPARATION METHOD THEREFOR**

(30) Priority: 18.01.2019 CN 201910049843; 11.07.2019 CN 201910625009
(71) Applicant: Chia Tai Tianqing Pharmaceutical Group Co., Ltd., Lianyungang, Jiangsu 222062 (CN)
(72) Inventor: WU, Lingyun, Shanghai 200131 (CN); LIU, Xile, Shanghai 200131 (CN); DING, Charles Z., Shanghai 200131 (CN); CHEN, Shuhui, Shanghai 200131 (CN); HU, Lihong, Shanghai 200131 (CN); ZHAO, Lele, Shanghai 200131 (CN); PAN, Wei, Shanghai 200131 (CN); HU, Guoping, Shanghai 200131 (CN); LI, Jian, Shanghai 200131 (CN); ZHAO, Ning, Shanghai 200131 (CN); ZHAO, Jun, Shanghai 201315 (CN)
(74) Representative: Angerhausen, Christoph
(86) International application number: PCT/CN2020/072767
(87) International publication number: WO 2020/147838

(57) **Abstract**

Provided are a salt of an EGFR inhibitor, a crystal form thereof and a preparation method therefor, and an application of the salt and the crystal form in the preparation of a treatment for non-small cell lung cancer. The salt has a structure as shown in formula (II).

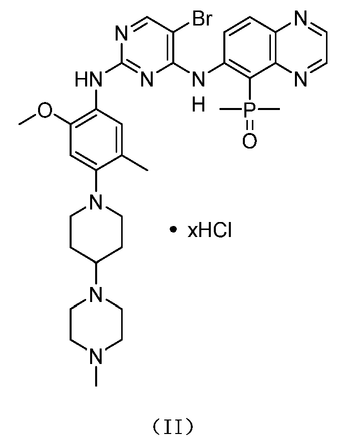

## Description

### CROSS-REFERENCE TO RELATED APPLICATIONS

The present application claims the benefit and priority to the Chinese Patent Application No. CN201910049843.3 filed with the China National Intellectual Property Administration on January 18, 2019 and the Chinese Patent Application No. CN201910625009.4 filed with the China National Intellectual Property Administration on July 11, 2019, which are incorporated herein by reference in their entirety.

### TECHNICAL FIELD

The present application relates to a salt of an EGFR inhibitor, a crystalline form and a preparation method thereof, and an application of the salt and the crystalline form in the preparation of a medicament for treating non-small cell lung cancer.

### BACKGROUND

Lung cancer is one of the most common malignant tumors worldwide, accounting for about 1.6 million new cases and 1.4 million deaths every year, in which cases of non-small cell lung cancer (NSCLC) accounts for about 80-85% of total lung cancer cases (information from the 10th Lung Cancer Summit).

The epidermal growth factor receptor tyrosine kinase inhibitor (EGFR-TKI), as a small molecule inhibitor, inhibits activation of tyrosine kinase by competing with an endogenous ligand for binding to EGFR, to block the EGFR signaling pathway, finally producing a series of biological effects such as the inhibition of proliferation and metastasis of tumor cells, the promotion of apoptosis of tumor cells and the like. Therefore, the EGFR-TKI is one of the main targets for treating lung cancer.

Although Osimertinib (AZD9291), a third-generation EGFR-TKI targeted drug, has a higher response rate for the drug resistance caused by T790M mutation, patients may still suffer from drug resistance. (Clin Cancer Res; 21(17), 2015). In 2015, analysis of drug resistance to AZD9291 in 15 patients was first reported (Nature Medicine, 21:560-562, 2015), in which the EGFR C797S mutation, the third mutation discovered, is one of the main mechanisms causing drug resistance to Osimertinib, and this mutation was found in about 40% of them. In addition, drug resistance to AZD9291 was also reported at some conferences. At the World Conference on Lung Cancer (WCLC 2015), Oxnard GR reported analysis of drug resistance in 67 patients, in which the cases with C797S mutation account for about 22%. At the American Society of Clinical Oncology (ASCO 2017), Piotrowska reported analysis of drug resistance in 23 patients and the cases with C797S mutation account for 22%; and Caicun Zhou et al. reported mechanism analysis of drug resistance in 99 patients, among them, the cases with C797S mutation also account for 22%. Therefore, it is greatly significant to overcome the drug resistance to AZD9291 caused by C797S mutation and provide a safer and more efficient fourth-generation EGFR C797S/T790M inhibitor for patients.

In an article (Nature, 534:129-132, 2016), it was reported that a compound, EAI045, is capable of overcoming the drug resistance to Osimertinib caused by C797S mutation. EAI045 is an allosteric inhibitor, and shows a good tumor inhibitory effect in the *in vivo* drug effect model of mice with L858R/T790M/C797S mutation when used in combination with an EGFR monoclonal antibody drug such as Cetuximab. However, this compound failed to be investigated in clinical research. In an article (Nature Communications, 8:14768, 2017), it was reported that the drug resistance to the third-generation targeted drug Osimertinib caused by C797S mutation can be overcome by combination therapy of Brigatinib (AP26113) and an EGFR monoclonal antibody (e.g. Cetuximab), and the result of the PC9 (EGFR-C797S/T790M/del19) drug effect model of mice shows that the combination therapy of Brigatinib with Panitumumab or Cetuximab exhibits good anti-tumor efficacy.

### SUMMARY

The present application provides a compound of formula (II): 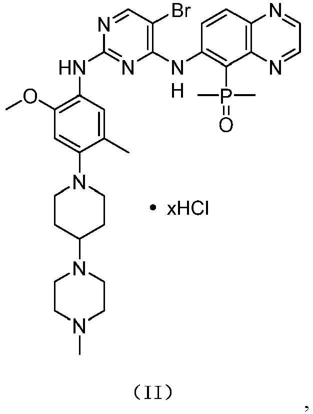 wherein x is selected from 3.0-5.0.
In some embodiments of the present application, the x is selected from the group consisting of 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9 and 5.0 or from a range formed by any of the above values. In some specific embodiments, the x is selected from 3.2-4.8, preferably 3.5-4.5, more preferably 3.8-4.2, and further preferably 4.0.

The present application provides a solid form of the compound of formula (II).

In some embodiments of the present application, the solid form of the compound of formula (II) is selected from the group consisting of amorphous form and crystalline form.

In some embodiments of the present application, crystalline form A has diffraction peaks at the following 2θ of 5.95°, 9.51° and 19.04°; typically has diffraction peaks at the following 2θ of 5.95°, 9.51°, 14.91°, 19.04°, 24.95° and 25.39°; more typically has diffraction peaks at the following 2θ of 5.95°, 9.51°, 14.91°, 16.48°, 19.04°, 23.76°, 24.95°, 25.39° and 27.08°; further typically has diffraction peaks at the following 2θ of 5.95°, 9.51°, 9.65°, 14.91°, 16.48°, 19.04°, 19.36°, 23.76°, 24.95°, 25.39° and 27.08°; and further more typically has diffraction peaks at the following 2θ of 5.95°, 9.51°, 9.65°, 13.61°, 14.91°, 16.48°, 19.04°, 19.36°, 23.76°, 24.95°, 25.39°, 27.08° and 31.88° in the X-ray powder diffraction pattern thereof.

**Table 1. XRPD diffraction peaks of crystalline form A of the compound of formula (II)**

| **Nos.** | **2θ (°)** | **Relative intensity (%)** | **Nos.** | **2θ (°)** | **Relative intensity (%)** |
|---|---|---|---|---|---|
| 1 | 5.95 | 44.4 | 16 | 21.69 | 12.5 |
| 2 | 8.81 | 11.3 | 17 | 23.32 | 10.3 |
| 3 | 9.51 | 81.4 | 18 | 23.76 | 27.2 |
| 4 | 9.65 | 69.5 | 19 | 24.60 | 11.6 |
| 5 | 12.89 | 19.2 | 20 | 24.95 | 44.0 |
| 6 | 13.61 | 22.0 | 21 | 25.39 | 43.6 |
| 7 | 14.91 | 37.7 | 22 | 26.01 | 11.6 |
| 8 | 15.37 | 12.2 | 23 | 26.27 | 12.8 |
| 9 | 15.78 | 14.1 | 24 | 27.08 | 21.2 |
| 10 | 16.48 | 24.1 | 25 | 29.45 | 14.1 |
| 11 | 17.62 | 15.7 | 26 | 29.67 | 10.6 |
| 12 | 19.04 | 100.0 | 27 | 30.07 | 11.9 |
| 13 | 19.36 | 35.4 | 28 | 31.50 | 14.6 |
| 14 | 19.48 | 16.0 | 29 | 31.88 | 21.4 |
| 15 | 20.42 | 17.6 | | | |

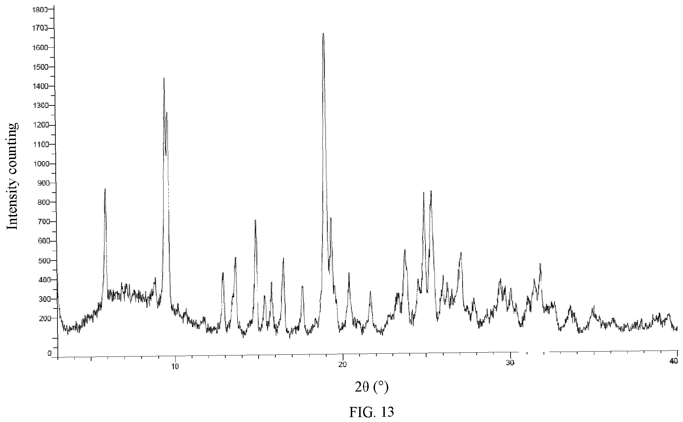

In some embodiments of the present application, the aforementioned crystalline form A has an XRPD pattern as shown in FIG. 13.

The present application provides a method for preparing crystalline form A of the compound of formula (II) comprising: dissolving the compound of formula (II) in a solvent, stirring and separating to give crystalline form A, wherein the solvent is selected from the group consisting of ethanol, isopropanol, tetrahydrofuran, acetonitrile, *n*-heptane, ethyl acetate, and acetone, and a mixture of any two or more of the above solvents. In some embodiments, the solvent is selected from ethanol.

The present application also provides a method for preparing crystalline form A of the compound of formula (II) comprising:
(a) adding the compound of formula (I) in a solvent, and heating the resulting solution up to 70-80 °C;
(b) dissolving hydrochloric acid in a solvent, slowly adding with the solution prepared in step (a), and stirring at 70-80 °C for 1-2 h; and
(c) cooling the solution to 20-30 °C, stirring for 12 h, filtering under reduced pressure and N₂ atmosphere protection, and drying;
wherein the solvent is ethanol, isopropanol, tetrahydrofuran, acetonitrile, *n*-heptane, ethyl acetate, acetone, or a mixture of any two or more of the above solvents.

The present application also provides crystalline form B of the compound of formula (II), wherein the crystalline form B has diffraction peaks at the following 2θ of 6.39°, 12.39° and 14.18°; typically has diffraction peaks at the following 2θ of 6.39°, 12.39°, 13.03°±0.2°, 14.18°, 19.03° and 26.76°; more typically has diffraction peaks at the following 2θ of 6.39°±0.2°, 12.39°±02°, 13.03°±0.2°, 14.18°±0.2°, 18.52°±0.2°, 19.03°±02°, 26.09°±0.2° and 26.76°±0.2°; and further typically has diffraction peaks at the following 2θ of 6.39°, 7.05°, 12.39°, 13.03°, 14.18°, 16.64°, 18.52°, 19.03°, 26.09°and 26.76°, in the X-ray powder diffraction pattern thereof.

The present application also provides crystalline form B of the compound of formula (II), wherein crystalline form B has diffraction peaks at the following 2θ of 6.39°, 12.39° and 14.18°; typically has diffraction peaks at the following 2θ of 6.39°, 12.39°, 13.03°, 14.18°, 19.03° and 26.76°; more typically has diffraction peaks at the following 2θ of 6.39°, 12.39°, 13.03°, 14.18°, 18.52°, 19.03°, 26.09° and 26.76°; and further typically has diffraction peaks at the following 2θ of 6.39°, 7.05°, 12.39°, 13.03°, 14.18°, 16.64°, 18.52°, 19.03°, 26.09° and 26.76°, in the X-ray powder diffraction pattern thereof.

In some embodiments of the present application, the XRPD pattern of the aforementioned crystalline form B has diffraction peaks as shown in Table 2.

**Table 2. XRPD diffraction peaks of crystalline form B of the compound of formula (II)**

| **Nos.** | **2θ (°)** | **Relative intensity (%)** | **Nos.** | **2θ (°)** | **Relative intensity (%)** |
|---|---|---|---|---|---|
| 1 | 4.75 | 16.9 | 16 | 24.11 | 14.0 |
| 2 | 6.39 | 26.8 | 17 | 24.73 | 6.8 |
| 3 | 7.05 | 13.4 | 18 | 25.42 | 15.8 |
| 4 | 12.39 | 39.9 | 19 | 26.09 | 73.2 |
| 5 | 13.03 | 26.2 | 20 | 26.76 | 71.3 |
| 6 | 14.18 | 100.0 | 21 | 27.20 | 14.0 |
| 7 | 15.72 | 4.0 | 22 | 27.96 | 17.9 |
| 8 | 16.64 | 27.4 | 23 | 28.64 | 7.1 |
| 9 | 18.52 | 58.5 | 24 | 29.15 | 27.1 |
| 10 | 19.03 | 56.4 | 25 | 30.08 | 11.2 |
| 11 | 20.00 | 13.7 | 26 | 30.67 | 11.4 |
| 12 | 21.06 | 16.7 | 27 | 31.12 | 8.7 |
| 13 | 22.57 | 15.5 | 28 | 31.53 | 12.7 |
| 14 | 23.00 | 4.7 | 29 | 33.35 | 21.6 |
| 15 | 23.67 | 19.4 | 30 | 33.94 | 12.3 |

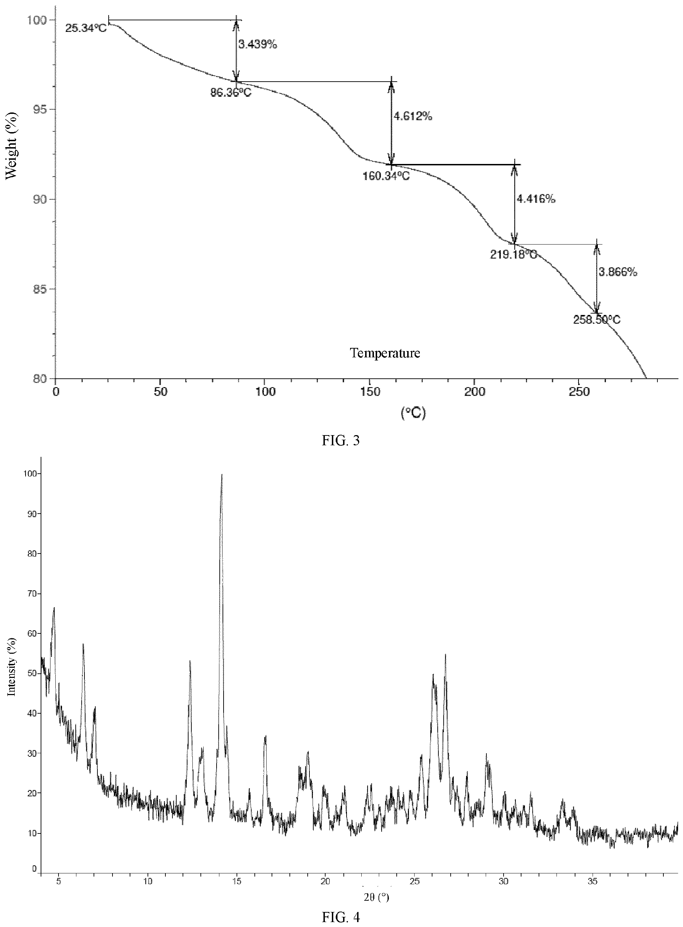

In some embodiments of the present application, the aforementioned crystalline form B has an XRPD pattern as shown in FIG. 4.

In some embodiments of the present application, the differential scanning calorimetry (DSC) curve of the aforementioned crystalline form B has starting points of endothermic peaks at 32.91 °C, 85.84 °C, 156.46 °C, 198.71 °C, and 261.12 °C. In some embodiments of the present application, the DSC curve of the aforementioned crystalline form B has a starting point of an exothermic peak at 283.64 °C.

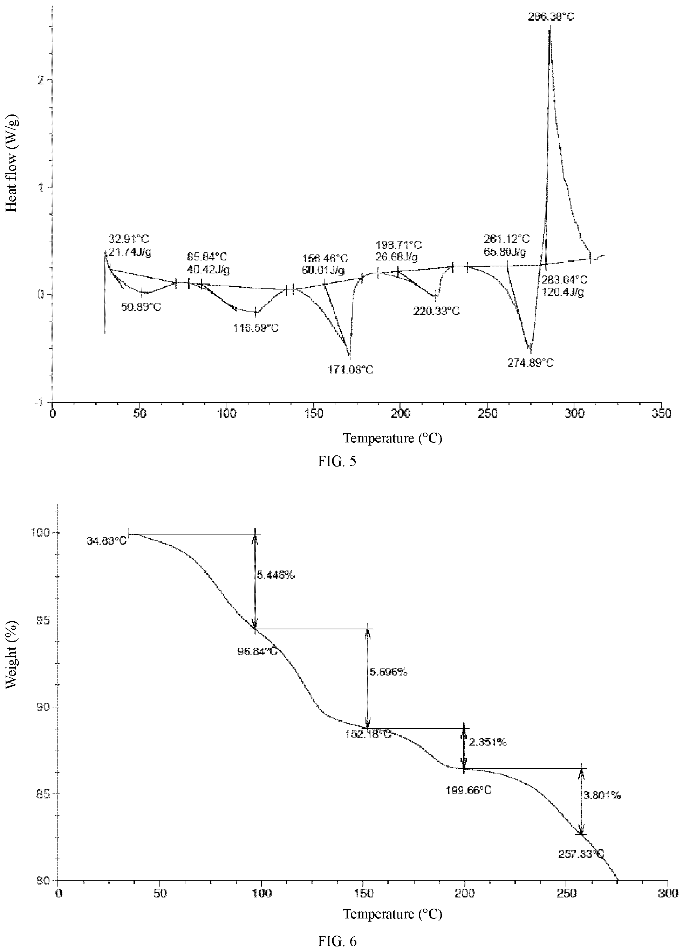

In some embodiments of the present application, the DSC pattern of the aforementioned crystalline form B is shown in FIG. 5.

In some embodiments of the present application, the thermogravimetric analysis (TGA) curve of the aforementioned crystalline form B shows a weight loss of 5.446% at 96.84 °C, 11.14% at 152.18 °C, 13.49% at 199.66 °C and 17.29% at 257.33 °C.

In some embodiments of the present application, the TGA pattern of the aforementioned crystalline form B is shown in FIG. 6.

In some embodiments of the present application, a method for preparing crystalline form B of the compound of formula (II) is provided, comprising: mixing the aforementioned compound of formula (II) with methanol, and separating to give crystalline form B.

The present application also provides a method for preparing crystalline form B of the compound of formula (II) comprising:
(a) adding the compound of formula (II) to a solvent to form a suspension;
(b) stirring the suspension at 35-45 °C for 24 h; and
(c) drying at 30-40 °C for 8-16 h after centrifugation;
wherein the solvent is methanol.

In some embodiments of the present application, a method for preparing crystalline form B of the compound of formula (II) is provided, comprising: mixing the aforementioned crystalline form A with methanol, and separating to give crystalline form B.

The present application also provides crystalline form C of the compound of formula (II), wherein crystalline form C has diffraction peaks at the following 2θ of 6.78°, 10.33° and 14.04°; typically has diffraction peaks at the following 2θ of 6.78°, 10.33°, 14.04°, 18.66°, 20.37° and 25.66°; more typically has diffraction peaks at the following 2θ of 6.78°, 10.33°, 14.04°, 18.66°, 20.37°, 25.09°, 25.66° and 26.62°; and further typically has diffraction peaks at the following 2θ of 6.78°, 10.33°, 13.65°, 14.04°, 18.66°, 20.37°, 21.04°, 25.09°, 25.66° and 26.62°, in the X-ray powder diffraction pattern thereof.

In some embodiments of the present application, the XRPD pattern of the aforementioned crystalline form C has diffraction peaks as shown in Table 3.

**Table 3. XRPD diffraction peaks of crystalline form C of the compound of formula (II)**

| **Nos.** | **2θ (°)** | **Relative intensity (%)** | **Nos.** | **2θ (°)** | **Relative intensity (%)** |
|---|---|---|---|---|---|
| 1 | 6.78 | 33.3 | 14 | 21.83 | 3.2 |
| 2 | 8.03 | 3.8 | 15 | 23.40 | 3.5 |
| 3 | 10.33 | 37.2 | 16 | 24.03 | 4.2 |
| 4 | 12.19 | 4.1 | 17 | 25.09 | 63.4 |
| 5 | 13.65 | 53 | 18 | 25.66 | 100 |
| 6 | 14.04 | 96.1 | 19 | 26.62 | 50.6 |
| 7 | 14.36 | 64.8 | 20 | 29.78 | 6.2 |
| 8 | 15.52 | 16.3 | 21 | 31.30 | 3 |
| 9 | 17.97 | 14.6 | 22 | 32.44 | 10.8 |
| 10 | 18.66 | 20.2 | 23 | 33.29 | 25 |
| 11 | 19.76 | 18 | 24 | 33.98 | 21 |
| 12 | 20.37 | 18.2 | 25 | 35.19 | 14.1 |
| 13 | 21.04 | 13.2 | | | |

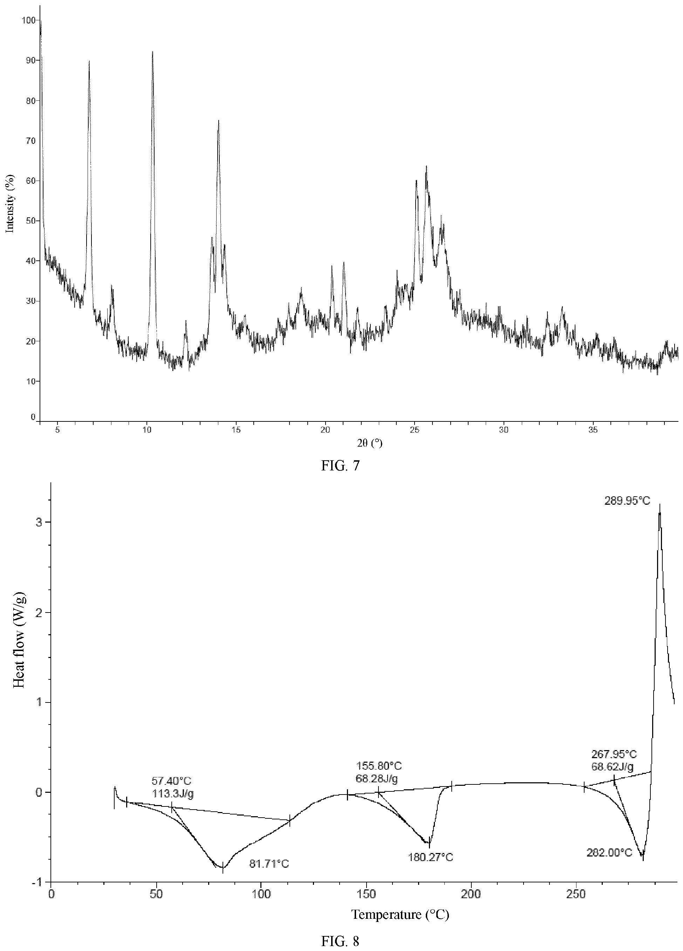

In some embodiments of the present application, the aforementioned crystalline form C has an XRPD pattern as shown in FIG. 7.

In some embodiments of the present application, the DSC curve of the aforementioned crystalline form C has starting points of endothermic peaks at 57.40 °C, 155.80 °C, and 267.95 °C. In some embodiments of the present application, the DSC curve of the aforementioned crystalline form C has an exothermic peak at 289.95 °C.

In some embodiments of the present application, the DSC pattern of the aforementioned crystalline form C is shown in FIG. 8.

In some embodiments of the present application, the TGA curve of the aforementioned crystalline form C shows a weight loss of 5.839% at 88.39 °C, 9.810% at 156.66 °C and 13.35% at 263.66 °C.

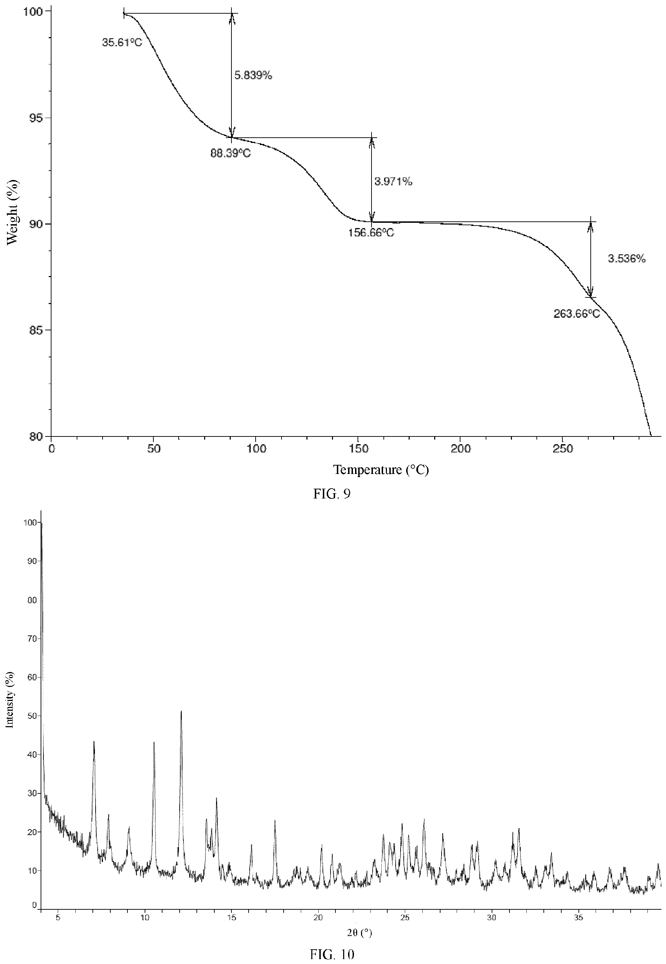

In some embodiments of the present application, the TGA pattern of the aforementioned crystalline form C is shown in FIG. 9.

The present application provides a method for preparing crystalline form C of the compound of formula (II) comprising: mixing the compound of formula (II) with a solvent, and separating to give crystalline form C of the compound of formula (II), wherein the solvent is selected from the group consisting of methanol, ethanol and a mixed solvent of isopropanol and water.

The present application provides a method for preparing crystalline form C of the compound of formula (II) comprising: mixing crystalline form A of the compound of formula (II) with a solvent, and separating to give crystalline form C of the compound of formula (II), wherein the solvent is selected from the group consisting of methanol, ethanol and a mixed solvent of isopropanol and water.

The present application also provides a method for preparing crystalline form C of the compound of formula (II) comprising:
(a) adding the compound of formula (II) to a solvent to form a suspension;
(b) stirring the suspension at 70-80 °C for 1-2 h, the suspension becoming clear during stirring;
(b) cooling the suspension to 20-30 °C with stirring for 12 h, a solid being precipitated during cooling; and
(c) drying the solid at 30-40 °C for 8-16 h after centrifugation;
wherein the solvent is selected from a mixed solvent of an alcohol and water, and the volume ratio of the alcohol to water in the mixed solvent is 10:1-1:1, excluding ethanol:water = 5:1 (v/v).

In some embodiments of the present application, the alcohol used in the method for preparing the aforementioned crystalline form C is selected from the group consisting of methanol, ethanol, n-propanol and isopropanol.

In some embodiments of the present application, the volume ratio of the alcohol to water used in the method for preparing the aforementioned crystalline form C is 10:1, 9:1, 8:1, 7:1, 6:1, 5:1, 4:1, 3:1, 2:1, 1:1, or a range formed by any of the above ratios.

The present application also provides crystalline form D of the compound of formula (II), wherein crystalline form D has diffraction peaks at the following 2θ of 7.08°, 10.53° and 12.10°; typically has diffraction peaks at the following 2θ of 7.08°, 9.11°, 10.53°, 12.10°, 13.78° and 17.51°; more typically has diffraction peaks at the following 2θ of 7.08°, 9.11°, 10.53°, 12.10°, 13.78°, 17.51°, 20.21° and 24.18°; and further more typically has diffraction peaks at the following 2θ of 7.08°, 7.92°, 9.11°, 10.53°, 12.10°, 13.78°, 16.15°, 17.51°, 20.21°, 24.18° and 26.11°, in the X-ray powder diffraction pattern thereof.

In some embodiments of the present application, the XRPD pattern of the aforementioned crystalline form D has diffraction peaks as shown in Table 4.

**Table 4. XRPD diffraction peaks of crystalline form D of the compound of formula (II)**

| **Nos.** | **2θ (°)** | **Relative intensity (%)** | **Nos.** | **2θ (°)** | **Relative intensity (%)** |
|---|---|---|---|---|---|
| 1 | 7.08 | 58.0 | 21 | 25.22 | 45.9 |
| 2 | 7.92 | 14.6 | 22 | 25.70 | 48.4 |
| 3 | 9.11 | 36.1 | 23 | 26.11 | 83.8 |
| 4 | 10.53 | 61.6 | 24 | 27.18 | 26.2 |
| 5 | 12.10 | 100.0 | 25 | 28.40 | 21.9 |
| 6 | 13.78 | 91.1 | 26 | 29.19 | 40.7 |
| 7 | 14.14 | 71.1 | 27 | 30.23 | 29.0 |
| 8 | 14.87 | 17.1 | 28 | 30.76 | 35.6 |
| 9 | 16.15 | 20.0 | 29 | 31.22 | 63.7 |
| 10 | 17.51 | 35.7 | 30 | 31.57 | 78.6 |
| 11 | 18.77 | 30.9 | 31 | 32.52 | 16.9 |
| 12 | 19.40 | 18.6 | 32 | 33.07 | 29.1 |
| 13 | 20.21 | 22.5 | 33 | 33.45 | 42.5 |
| 14 | 20.81 | 21.3 | 34 | 34.36 | 12.6 |
| 15 | 21.24 | 21.1 | 35 | 35.42 | 12.7 |
| 16 | 22.20 | 6.4 | 36 | 35.90 | 18.9 |
| 17 | 23.25 | 22.5 | 37 | 36.86 | 24.8 |
| 18 | 23.76 | 40.1 | 38 | 37.71 | 31.1 |
| 19 | 24.18 | 56.1 | 39 | 39.05 | 10.3 |
| 20 | 24.83 | 54.1 | | | |

In some embodiments of the present application, the aforementioned crystalline form D has an XRPD pattern as shown in FIG. 10.

In some embodiments of the present application, the DSC curve of the aforementioned crystalline form D has starting points of endothermic peaks at 52.15 °C, 100.92 °C, 161.19 °C, and 265.11 °C. In some embodiments of the present application, the DSC curve for the aforementioned crystalline form D has an exothermic peak at 287.78 °C.

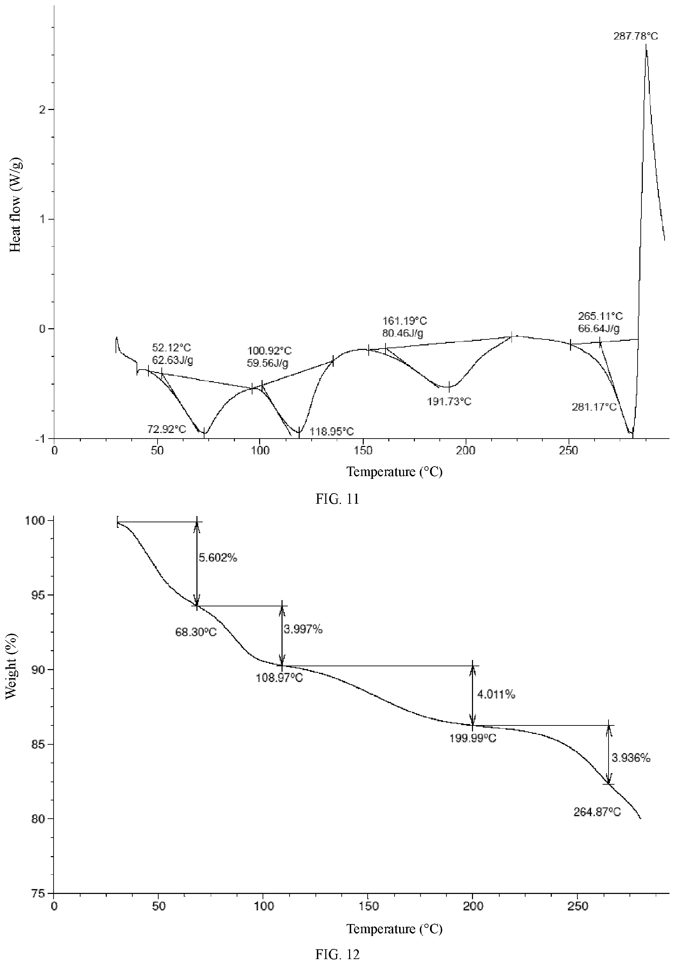

In some embodiments of the present application, the DSC pattern of the aforementioned crystalline form D is shown in FIG. 11.

In some embodiments of the present application, the TGA curve of the aforementioned crystalline form D shows a weight loss of 5.602% at 68.30 °C, 9.599% at 108.97 °C, 13.61% at 199.99 °C and 17.55% at 264.87 °C.

In some embodiments of the present application, the TGA pattern of the aforementioned crystalline form D is shown in FIG. 12.

The present application also provides a method for preparing crystalline form D of the compound of formula (II) comprising: mixing the compound of formula (II) with a solvent, and separating to give crystalline form D, wherein the solvent is selected from the group consisting of a mixed solvent of ethanol and water, and a mixed solvent of acetone and water, and the volume ratio of ethanol or acetone to water in the mixed solvent is 5:1.

A method for preparing the aforementioned crystalline form D of the compound of formula (II) comprises: mixing crystalline form A of the compound of formula (II) with a solvent, and separating to give crystalline form D, wherein the solvent is selected from the group consisting of a mixed solvent of ethanol and water, and a mixed solvent of acetone and water, and the volume ratio of ethanol or acetone to water in the mixed solvent is 5:1.

The present application also provides a method for preparing crystalline form D of the compound of formula (II) comprising:
(a) adding the compound of formula (II) to a solvent to form a suspension;
(b) stirring the suspension at 70-80 °C for 1-2 h, the suspension becoming clear during stirring;
(b) cooling the suspension to 20-30 °C with stirring for 12 h, a solid being precipitated during cooling; and
(c) drying the solid at 30-40 °C for 8-16 h after centrifugation;
wherein the solvent is selected from the group consisting of a mixed solvent of ethanol and water, and a mixed solvent of acetone and water, and the volume ratio of ethanol or acetone to water in the mixed solvent is 5:1.

In some embodiments of the present application, the characteristic diffraction peaks of the crystalline form A, crystalline form B, crystalline form C or crystalline form D at 2θ have an error of ±0.2° in the X-ray powder diffraction pattern.

In some embodiments of the present application, the starting points of the endothermic peaks of the crystalline form A, crystalline form B, crystalline form C or crystalline form D further preferably have an error of ± 2 °C in the DSC curve.

In some embodiments of the present application, the weight loss temperatures have an error of ±2 °C in the TGA curve.

The present application also provides a crystalline form composition of the crystalline form A, crystalline form B, crystalline form C or crystalline form D. In some embodiments of the present application, each of the crystalline form A, crystalline form B, crystalline form C or crystalline form D is 50wt% or more, preferably 80wt% or more, more preferably 90wt% or more, and most preferably 95wt% or more of the weight of the crystalline form composition.

The present application also provides a pharmaceutical composition of the compound of formula (II). The pharmaceutical composition comprises a therapeutically effective amount of the compound of formula (II), and furthermore, may or may not comprise a pharmaceutically acceptable carrier, excipient and/or vehicle.

The present application also provides a pharmaceutical composition of the compound of formula (II). The pharmaceutical composition comprises a therapeutically effective amount of the compound of formula (II) or a solid form, a crystalline form or a crystalline form composition thereof, and furthermore, may or may not comprise a pharmaceutically acceptable carrier, excipient and/or vehicle.

The present application also provides a compound of formula (II) for treating an EGFR-related disease.

The present application also provides use of the compound of formula (II) in the preparation of a medicament for treating an EGFR-related disease.

The present application also provides use of the compound of formula (II) in treating an EGFR-related disease.

The present application also provides a method for treating an EGFR-related disease, comprising administering a therapeutically effective amount of the compound of formula (II) to a patient in need.

The present application also provides the aforementioned compound of formula (II), or a solid form of the compound of formula (II), or a crystalline form of the compound of formula (II), or a crystalline form composition thereof, or a pharmaceutical composition thereof for use in treating an EGFR-related disease.

The present application also provides use of the aforementioned compound of formula (II), or a solid form of the compound of formula (II), or a crystalline form of the compound of formula (II), or a crystalline form composition thereof, or a pharmaceutical composition thereof in the preparation of a medicament for treating an EGFR-related disease.

The present application also provides use of the aforementioned compound of formula (II), or a solid form of the compound of formula (II), or a crystalline form of the compound of formula (II), or a crystalline form composition thereof, or a pharmaceutical composition thereof in treating an EGFR-related disease.

The present application also provides a method for treating an EGFR-related disease, comprising administering a therapeutically effective amount of the aforementioned compound of formula (II), or a solid form of the compound of formula (II), or a crystalline form of the compound of formula (II), or a crystalline form composition thereof, or a pharmaceutical composition thereof to a patient in need.

In some embodiments of the present application, the EGFR-related disease is selected from lung cancer.

### Technical Effects

The crystalline forms according to the present application have good stability under the conditions of illumination, high temperature and high humidity, and good pharmaceutical properties, and can be used in the preparation of medicaments.

### Definitions and Description

Unless otherwise stated, the following terms and phrases used herein are intended to have the following meanings. A particular phrase or term, unless otherwise specifically defined, should not be considered as uncertain or unclear, but should be construed according to its common meaning. When referring to a trade name herein, it is intended to refer to its corresponding commercial product or its active ingredient.

The word "comprise" and variations thereof, such as "comprises" or "comprising", will be understood in an open, non-exclusive sense, i.e., "including but not limited to".

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt", as a pharmaceutically acceptable salt, for example, may refer to a metal salt, an ammonium salt, a salt formed with an organic base, a salt formed with an inorganic acid, a salt formed with an organic acid, a salt formed with a basic or acidic amino acid, and the like.

The term "pharmaceutically acceptable excipient" refers to an inert substance administered with an active ingredient to facilitate administration of the active ingredient, including, but not limited to, any glidant, sweetener, diluent, preservative, dye/coloring agent, flavor enhancer, surfactant, wetting agent, dispersant, disintegrant, suspending agent, stabilizer, isotonizing agent, solvent or emulsifier acceptable for use in humans or animals (e.g., domesticated animals) as permitted by the National Medical Products Administration. Non-limiting examples of the excipients include calcium carbonate, calcium phosphate, various sugars and various types of starch, cellulose derivatives, gelatin, vegetable oils, and polyethylene glycols.

The term "pharmaceutical composition" refers to a mixture consisting of one or more of the compounds or salts thereof accoring to the present application and a pharmaceutically acceptable excipient. The pharmaceutical composition is intended to facilitate the administration of the compound to an organism.

The pharmaceutical composition of the present application can be prepared by combining the compound of the present application with a suitable pharmaceutically acceptable excipient, and can be formulated, for example, into a solid, semisolid, liquid, or gaseous formulation, such as tablet, pill, capsule, powder, granule, ointment, emulsion, suspension, suppository, injection, inhalant, gel, microsphere, and aerosol.

Typical routes of administration of the crystalline forms or the pharmaceutical composition thereof described in the present application include, but are not limited to, oral, rectal, topical, inhalation, parenteral, sublingual, intravaginal, intranasal, intraocular, intraperitoneal, intramuscular, subcutaneous and intravenous administration. The pharmaceutical composition of the present application can be manufactured by methods well known in the art, such as conventional mixing, dissolving, granulating, dragee-making, levigating, emulsifying, and lyophilizing.

In some embodiments, the pharmaceutical composition is in an oral form. For oral administration, the pharmaceutical composition can be formulated by mixing the active compounds with pharmaceutically acceptable excipients well known in the art. These excipients enable the compounds of the present application to be formulated into tablets, pills, pastilles, dragees, capsules, liquids, gels, slurries, suspensions and the like for oral administration to a patient.

Therapeutic dosages of the compound of the present application may be determined, for example, depending on the specific use of a treatment, the route of administration of the compound, the health and condition of a patient, and the judgment of a prescribing physician. The proportion or concentration of the compound of the present application in a pharmaceutical composition may not be constant and depends on a variety of factors including dosages, chemical properties (e.g., hydrophobicity), and routes of administration. The term "treating" means administering the compound or formulation described in the present application to ameliorate or eliminate a disease or one or more symptoms associated with the disease, and includes:
(i) inhibiting a disease or disease state, i.e., arresting its development; and
(ii) alleviating a disease or disease state, i.e., causing regression of the disease or disease state.

The term "preventing" means administering the compound or formulation described in the present application to prevent a disease or one or more symptoms associated with the disease, and includes: preventing the occurrence of the disease or disease state in a mammal, particularly when such a mammal is predisposed to the disease state but has not yet been diagnosed as having it.

For drugs and pharmacological active agents, the term "therapeutically effective amount" refers to an amount of a drug or a medicament that is sufficient to provide the desired effect but is non-toxic. The determination of the effective amount varies from person to person, depending on the age and general condition of a subject and also depending on the particular active substance. The appropriate effective amount in a case may be determined by those skilled in the art in the light of routine tests.

The therapeutically effective amount of the crystalline forms described in the present application is from about 0.0001 to 20 mg/kg body weight (bw)/day, for example from 0.001 to 10 mg/kg bw/day.

The dosage frequency of the crystalline forms described in the present application depends on needs of an individual patient, e.g., once or twice daily, or more times daily. Administration may be intermittent, for example, in a period of several days, the patient receives a daily dose of the crystalline forms, and in the following period of several days or more days, the patient does not receive the daily dose of the crystalline forms.

The intermediate compounds described in the present application can be prepared by a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present application.

The chemical reactions of the particular embodiments according to the present application are carried out in a suitable solvent that must be suitable for the chemical changes in the present application and the reagents and materials required therefor. In order to acquire the compounds of the present application, it is sometimes necessary for those skilled in the art to modify or select a synthesis procedure or a reaction scheme based on the existing embodiments.

The present application is described in detail below by way of examples, which are not intended to limit the present application in any way.

All solvents used herein are commercially available and can be used without further purification, and supplier's catalog names are given for commercially available compounds.

The following abbreviations are used in the present application: DIEA represents *N*,*N*-diisopropylethylamine; HPLC represents high performance liquid chromatography; TLC represents thin layer chromatography.

It should be noted that in the powder X-ray diffraction spectrum, the position and relative intensity of a peak may vary due to measuring instruments, measuring methods/conditions, and other factors. For any specific crystalline form, the position of a peak may have an error, and the measurement of 2θ may have an error of ±0.2°. Therefore, this error should be considered when determining each crystalline form, and crystalline forms within this margin of error are within the scope of the present application.

It should be noted that, for the same crystalline form, the position of an endothermic peak in the DSC pattern may vary due to measuring instruments, measuring methods/conditions, and other factors. For any specific crystalline form, the position of an endothermic peak may have an error of ±2 °C. Therefore, this error should be considered when determining each crystalline form, and crystalline forms within this margin of error are within the scope of the present invention.

It should be noted that, for the same crystalline form, the position of a weight loss temperature in the TGA pattern may vary due to measuring instruments, measuring methods/conditions, and other factors. For any specific crystalline form, the weight loss temperature may have an error of ±2 °C. Therefore, this error should be considered when determining each crystalline form, and crystalline forms within this margin of error are within the scope of the present application.

It should be noted that, in the preparation of a pharmaceutical crystalline form, it is almost inevitable that solvent molecules and compound molecules may form cocrystals and remain in solids when they are in contact, thereby forming solvates, including stoichiometric solvates and non-stoichiometric solvates. All of the solvates are included within the scope of the present application.

### 1. Instruments and analytical methods

### 1.1. X-ray powder diffraction (X-ray Powder Diffractometer (XRPD))

Instrument model: Brook D8 advance X-ray diffractometer;
Test conditions: the detailed XRPD parameters are as follows: X-ray generator: Cu, Kα, (λ = 1.54056Ǻ); tube voltage: 40 kV; tube current: 40 mA; scatter slit: 0.60 mm; detector slit: 10.50 mm; anti-scatter slit: 7.10 mm; scan range: 3-40°; step size: 0.02°; step time: 0.12 s; rotation speed of sample plate: 15 rpm.

### 1.2. Differential thermal analysis (Differential Scanning Calorimeter (DSC))

Instrument model: TA Q2000 differential scanning calorimeter;
Test conditions: placing 0.5-1 mg of the sample in a DSC aluminum pan for testing; method: from room temperature to 250 °C at a heating rate of 10 °C/min.

### 1.3. Thermogravimetric analysis (Thermal Gravimetric Analyzer (TGA))

Instrument model: TA Q5000IR thermogravimetric analyzer;
Test conditions: placing 2-5 mg of the sample in a TGA platinum pan for testing; method: from room temperature to 300 °C at a heating rate of 10 °C/min.

### 1.4. Ion chromatography detection of chloride ions

Instrument model: Shimadzu LC-20AD sp

**Test conditions:**

| | |
|---|---|
| Software version | Lab Solution Version 5.92 |
| Chromatography column | Shimadzu Shim-pack IC-A3 4.6MM*15cm |
| Mobile phase | 8.0 mmol/L 4-hydroxybenzoic acid+3.2 mmol/L Bis-Tris |
| Column temperature | 40 °C |
| Flow rate | 1.5 mL/min |
| Detector | CDD-10A VP |
| Run time of liquid phase | 18 min |

### BRIEF DESCRIPTION OF THE DRAWINGS

FIG. 1 is an XRPD pattern of crystalline form A of the compound of formula (IIA) in Example 2A.
FIG. 2 is a DSC pattern of crystalline form A of the compound of formula (IIA) in Example 2A.
FIG. 3 is a TGA pattern of crystalline form A of the compound of formula (IIA) in Example 2A.
FIG. 4 is an XRPD pattern of crystalline form B of the compound of formula (IIA).
FIG. 5 is a DSC pattern of crystalline form B of the compound of formula (IIA).
FIG. 6 is a TGA pattern of crystalline form B of the compound of formula (IIA).
FIG. 7 is an XRPD pattern of crystalline form C of the compound of formula (IIA).
FIG. 8 is a DSC pattern of crystalline form C of the compound of formula (IIA).
FIG. 9 is a TGA pattern of crystalline form C of the compound of formula (IIA).
FIG. 10 is an XRPD pattern of crystalline form D of the compound of formula (IIA).
FIG. 11 is a DSC pattern of crystalline form D of the compound of formula (IIA).
FIG. 12 is a TGA pattern of crystalline form D of the compound of formula (IIA).
FIG. 13 is an XRPD pattern of crystalline form A of the compound of formula (IIA) in Example 2B.

### DETAILED DESCRIPTION

In order to better understand the content of the present application, further description is given with reference to specific examples, but the specific embodiments are not intended to limit the content of the present application.

### Example 1: Preparation of the Compound of Formula (I)

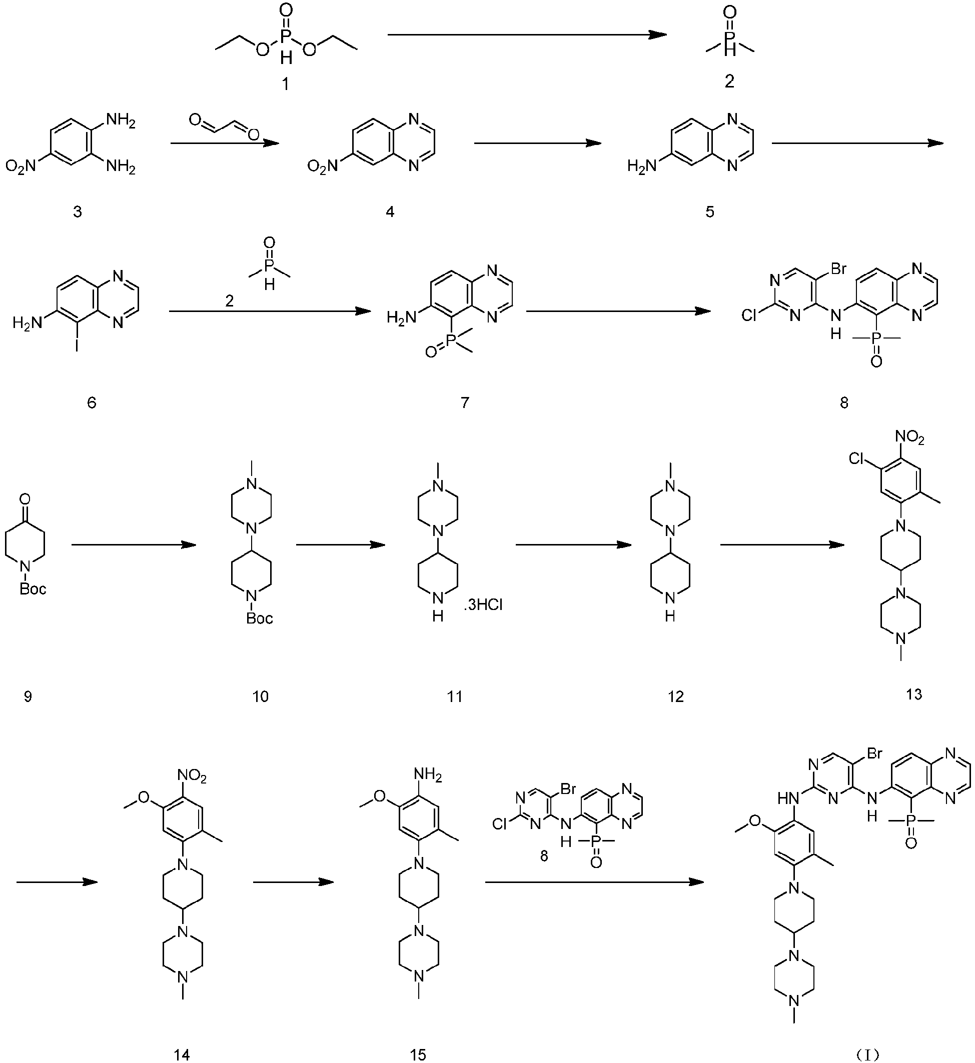

### Step 1:

Compound 1 (500 g, 3.62 mol, 1.0 eq) was dissolved in tetrahydrofuran (3.75 L), methyl magnesium bromide (10.86 mol, 3.62 L, 3.0 eq) was added dropwise to the above solution, and the reaction system was maintained at around 10 °C. After the dropwise addition, the reaction system was stirred at 25 °C for 12 h. After the reaction was completed as detected by TLC, the reaction solution was added to a saturated aqueous potassium carbonate solution (1.48 kg of potassium carbonate dissolved in 1.8 L of water), then the resulting mixture was slurried with ethanol and filtered, and the filtrate was collected and concentrated to give compound 2 (crude), which was directly used in the next step. ¹HNMR (400 MHz, deuterated dimethyl sulfoxide) δ = 7.57-7.54 (m, 0.5H), 6.48-6.40 (m, 0.5H), 1.51 (d, *J* = 4.0 Hz, 3H), 1.48 (d, *J* = 4.0 Hz, 3H).

### Step 2:

Compound 3 (534 g, 3.49 mol, 1.0 eq) was added to 3.4 L of ethanol, and the reaction solution was added dropwise with an aqueous glyoxal solution (607.12 g, 4.18 mol, 1.2 eq, 40% concentration) at 70 °C to 80 °C and then reacted at 75 °C for 3 h. After the reaction was completed as detected by HPLC, a large amount of solid was precipitated. The solid was filtered, and the filter cake was collected and dried to give compound 4. ¹H NMR (400 MHz, deuterated dimethyl sulfoxide) δ = 9.18 (s, 2H), 8.93 (d, *J* = 2.4 Hz, 1H), 8.58 (dd, *J* = 2.6, 9.2 Hz, 1H), 8.36 (d, *J* = 9.0 Hz, 1H).

### Step 3:

Compound 4 (200 g, 1.14 mol, 1.0 eq) was placed in methanol (2 L), and wet Pd/C (15 g) was added slowly under nitrogen atmosphere protection. The reaction system was evacuated and then purged with nitrogen once, and then the reaction system was evacuated and purged with hydrogen 3 times. Hydrogen was introduced to a pressure of 2 MPa. The reaction system was reacted at 20-30 °C for 12 h, and TLC showed completion of the reaction. After the reaction was completed, the reaction was filtered through celite, the filter cake was washed with methanol (1 L), and the filtrates were combined, and concentrated by rotary evaporation at 40-50 °C to precipitate a large amount of brown solid. After filtration under reduced pressure, the filter cake was dried in a vacuum drying oven at 40 °C to constant weight, to give compound 5. ¹H NMR (400 MHz, deuterated dimethyl sulfoxide) δ = 8.60 (d, *J* = 2.0 Hz, 1H), 8.45 (d, *J* = 2.0 Hz, 1H), 7.73 (d, *J* = 9.0 Hz, 1H), 7.24 (dd, *J* = 2.4, 9.0 Hz, 1H), 6.92 (d, *J* = 2.4 Hz, 1H), 6.07 (s, 2H).

### Step 4:

Compound 5 (441 g, 3.04 mol, 1.0 eq) was dissolved in dichloromethane (5 L) and methanol (0.5 L), sodium bicarbonate (331.78 g, 3.95 mol, 1.3 eq) was added under nitrogen atmosphere protection, and then iodine chloride (517.90 g, 3.19 mol, 1.05 eq) was slowly added dropwise to the reaction solution, and the reaction system was maintained at around 10 °C. After the reaction was completed as monitored by HPLC, the reaction solution was filtered, and the filtrate was concentrated under reduced pressure to give compound 6. ¹H NMR (400 MHz, deuterated dimethyl sulfoxide) δ = 8.72 (d, *J* = 2.0 Hz, 1H), 8.51 (d, *J* = 2.0 Hz, 1H), 7.77 (d, *J* = 9.0 Hz, 1H), 7.41 (d, *J* = 9.0 Hz, 1H).

### Step 5:

Compound 6 (760 g, 2.80 mol, 1.0 eq) and compound 2 (525 g, 3.36 mol, 1.2 eq) were dissolved in 1,4-dioxane (7.6 L) and then palladium acetate (31.47 g, 0.14 mol, 0.05 eq), Xantphos (81.12 g, 0.14 mol, 0.05 eq) and potassium phosphate (892.74 g, 4.21 mol, 1.5 eq) were added. The reaction system was warmed up to 100 °C and stirred under nitrogen atmosphere protection for 12 h, and the reaction was completed as monitored by HPLC. The reaction solution was diluted with dichloromethane (3.8 L) and filtered. The filtrate was collected and concentrated to give a crude product. The crude product was slurried with ethyl acetate to give compound 7. ¹H NMR (400 MHz, deuterated dimethyl sulfoxide) δ = 8.63 (d, *J* = 2.0 Hz, 1H), 8.49 (d, *J* = 2.2 Hz, 1H), 7.82 (d, *J* = 9.3 Hz, 1H), 7.23 (dd, *J* = 4.2, 9.3 Hz, 1H), 1.88 (d, *J* = 13.9 Hz, 6H).

### Step 6:

Compound 7 (500 g, 2.23 mol, 1.0 eq) was dissolved in *N*-methylpyrrolidinone (2.5 L) at room temperature under N₂ protection, 5-bromo-2,4-dichloropyrimidine (1016 g, 4.46 mol, 2.0 eq) and DIEA (466 mL, 2.68 mol, 1.2 eq) were added, and the reaction system was warmed up to 95 °C and stirred for 16 h. After the reaction was completed as monitored by HPLC, the reaction solution was cooled to room temperature. The reaction solution was poured into water, a large amount of solid was precipitated. After filtration, the filter cake was slurried with ethanol (1 L), washed with ethyl acetate (0.5 L), and dried in a vacuum drying oven at 50 °C to constant weight, to give compound 8. ¹HNMR (400 MHz, deuterated dimethyl sulfoxide) δ =13.26 (s, 1H), 8.94 (s, 2H), 8.84-8.81 (m, 1H), 8.64 (s, 1H), 8.29 (d, *J* = 9.2 Hz, 1H), 2.09 (s, 3H), 2.06 (s, 3H).

### Step 7:

Compound 9 (150 g, 0.75 mol, 1.0 eq) and 1-methylpiperazine (100 g, 0.99 mol, 1.33 eq) were added to methanol (1 L), Pd/C (15 g) was added under nitrogen atmosphere protection, and the reaction system was warmed up to 60 °C and stirred under hydrogen pressure (50 Pa) for 48 h. After the reaction was completed as monitored by HPLC, the reaction solution was cooled to 20-30 °C and filtered, the filtrate was concentrated to give a crude product, and the crude product was recrystallized from petroleum ether to give compound 10. ¹HNMR (400 MHz, deuterated chloroform) δ = 4.13 (s, 2H), 2.70-2.33 (m, 10H), 2.28 (s, 3H), 1.83-1.79 (m, 2H), 1.45 (s, 3H), 1.42-1.39 (m, 3H).

### Step 8:

Compound 10 (860 g, 3.03 mol, 1.0 eq) was dissolved in methanol (2 L) at room temperature, then hydrochloric acid methanol solution (4 mol, 7.59 L, 10 eq) was added and then the reaction system was stirred at room temperature for 48 h. After the reaction was completed as detected by TLC, the reaction solution was concentrated under reduced pressure to give compound 11. ¹H NMR (400 MHz, deuterated water) δ = 3.59-3.50 (m, 11H), 3.04 (t, *J* = 13.2 Hz, 2H), 2.94 (s, 3H), 2.36 (d, *J* = 13.6 Hz, 2H), 1.92-1.83 (m, 2H).

### Step 9:

Compound 11 (1.01 kg, 3.47 mol, 1.0 eq) was added to a solution of dichloromethane (5 L) and methanol (0.5 L) at room temperature, sodium hydroxide (429.86 g, 10.75 mol, 3.1 eq) was added under N₂ atmosphere protection, and the reaction solution was warmed up to 20-30 °C and stirred for 12 h. The reaction solution was filtered, and the filtrate was collected and concentrated to give compound 12. ¹H NMR (400 MHz, deuterated chloroform) δ = 3.12 (d, *J* = 12.4 Hz, 2H), 2.59-2.35 (m, 12H), 2.47 (s, 3H), 1.82 (d, *J* = 12 Hz, 2H), 1.39-1.33 (m, 2H).

### Step 10:

1-chloro-5-fluoro-4-methyl-2-nitrobenzene (1.68 kg, 8.86 mol, 1.0 eq) and compound 12 (1.79 kg, 9.75 mol, 1.1 eq) were dissolved in dimethyl sulfoxide (9 L) at 20 °C, and DIEA (1.26 kg, 9.75 mol, 1.1 eq) was added. After the addition was completed, the reaction solution was stirred at 60 °C for 20 h; after the reaction was completed as detected by HPLC, the reaction solution was poured into water (18 L), and then a large amount of yellow solid was precipitated. The reaction system was filtered, the filter cake was slurried with ethanol (3.6 L) and filtered, and the obtained filter cake was dried in vacuum at 40 °C to constant weight, to give compound 13. ¹H NMR (400 MHz, deuterated chloroform) δ = 7.75 (s, 1H), 6.91 (s, 1H), 3.25-3.22 (m, 2H), 2.67-2.42 (m, 11H), 2.44 (s, 3H), 2.42 (s, 3H), 1.92 (d, *J* = 12 Hz, 2H), 1.65-1.61 (m, 2H).

### Step 11:

Compound 13 (980.13 g, 2.78 mol, 1.0 eq) and *N*-methylpyrrolidone (5 L) were added sequentially into a reaction kettle, and the temperature in the reaction kettle was adjusted to 15-20 °C. Sodium methoxide (328.15 g, 6.07 mol, 2.2 eq) was added slowly in portions into the reaction kettle, and the reaction system was maintained at 15-25 °C. After the reaction was completed as monitored by HPLC, the reaction solution was added into distilled water (20 L) and maintained at 10-20 °C, and a large amount of solid was precipitated during adding. The solid was filtered, the filter cake was collected, slurried with ethanol and filtered. The filter cake was collected and dried in a vacuum drying oven to constant weight, to give compound 14. ¹H NMR (400 MHz, deuterated methanol) δ = 7.77 (s, 1H), 6.73 (s, 1H), 3.94 (s, 3H), 3.04 (m, 2H), 2.80-2.72 (m, 11H), 2.32 (s, 3H), 2.27 (s, 3H), 2.00-2.01 (m, 2H), 1.63-1.62 (m, 2H).

### Step 12:

Compound 14 (638.01 g, 1.83 mol, 1.0 eq), and methanol (6 L) were added sequentially into a high-pressure reaction kettle, and wet Pd/C (64.01 g) was added under argon atmosphere protection. The reaction system was purged with argon once, purged with hydrogen 3 times, and then subjected to reaction under hydrogen pressure (2 MPa) at a temperature of 20-40 °C. After the reaction was completed as monitored by HPLC, the solid was filtered, and the filtrate was collected. The reaction system was then transferred to a 50 L reaction kettle, added with thiourea resin (240.10 g) and stirred at 25 °C for 12 h under nitrogen atmosphere protection. The reaction solution was filtered, and the filtrate was collected. The filtrate was concentrated in a rotary evaporator under reduced pressure to give a solid, the solid was collected and dried in a vacuum drying oven to constant weight, to give compound 15. ¹H NMR (400 MHz, deuterated methanol) δ = 6.49 (s, 1H), 6.48 (s, 1H), 3.74 (s, 3H), 3.08-2.98 (m, 2H), 2.58-2.42 (m, 11H), 2.24 (s, 3H), 2.09 (s, 3H), 1.85-1.82 (m, 2H), 1.63-1.62 (m, 2H).

### Step 13:

Compound 15 (910.34 g, 2.91 mol, 1.2 eq), compound 8 (1000.06 g, 2.42 mol, 1.0 eq) and *N*-methylpyrrolidinone (5 L) were added in order into a reaction kettle. The reaction solution was stirred, added dropwise with methanesulfonic acid (722.14 g, 7.51 mol, 3.1 eq) at 20-40 °C, during which the temperature was maintained no higher than 40 °C. The reaction solution was warmed up to 85-90 °C. After the reaction was completed as monitored by HPLC, sodium hydroxide solution (1 mol, 15 L) was added into the reaction solution, and a large amount of solid was precipitated. The solid was filtered and the filter cake was collected. The filter cake was redissolved in ethanol (10 L), the reaction system was warmed to reflux, the addition of distilled water (20 L) was performed under heating reflux, and a large amount of solid was precipitated. The reaction solution was filtered, and the filter cake was collected. The collected filter cake was added into a reaction kettle, ethanol (15 L) and a solution of hydrochloric acid in ethanol (6 L) (2 L of concentrated hydrochloric acid dissolved in 4 L of ethanol) were added, and then the reaction system was heated for 2 h. Then the reaction system was naturally cooled to room temperature, and a large amount of solid was precipitated. The solid was collected by filtration and dissolved in distilled water (10 L), the solution was added with sodium hydroxide solution (1 mol, 10 L), a large amount of solid was precipitated, followed by stirring for 1 h. The solid was filtered, and the filter cake was collected and dried in a vacuum drying oven to constant weight, to give a compound of formula (I). ¹H NMR (400 MHz, deuterated methanol) δ = 8.94 (d, *J* = 4Hz, 1H), 8.92 (1H, br), 8.91 (s, 1H), 8.19 (s, 1H), 7.95 (d, *J* = 9.6 Hz, 1H), 7.59 (s, 1H), 6.72 (s, 1H), 3.84 (s, 3H), 3.16-3.13 (m, 2H), 2.70-2.37 (m, 11H), 2.31 (s, 3H), 2.16 (s, 3H), 2.12 (s, 3H), 2.06 (s, 3H), 2.02-1.99 (m, 2H); 1.71-1.68 (m, 2H).

### Example 2A: Preparation of Crystalline Form A of the Compound of Formula (IIA)

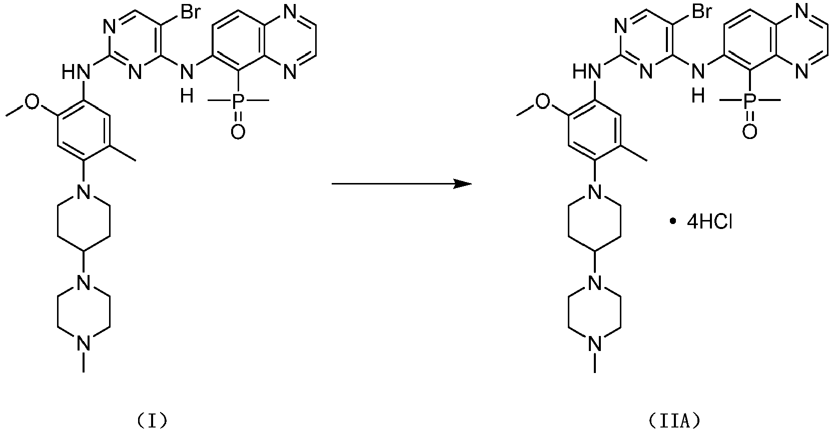

The compound of formula (I) (96 g, 131.16 mmol, 1 eq) was added into ethanol (500 mL) and the suspension was heated up to 78 °C. Concentrated hydrochloric acid (1.31 mol, 125.03 mL, 37.5% concentration, 10 eq) was diluted with ethanol (500 mL) and added dropwise to a reaction kettle under reflux, the reaction solution became clear when half amount of the diluted hydrochloric acid was added, and a large amount of solid was precipitated near the end of the addition. The reaction solution was stirred at 75-80 °C for 2 h, and then naturally cooled to 20-30 °C, and a large amount of off-white solid was precipitated, followed by stirring for 12 h. The solid was filtered and the filter cake was washed twice with ethanol. The filter cake was collected and dried at 40 °C in a vacuum oven to constant weight.

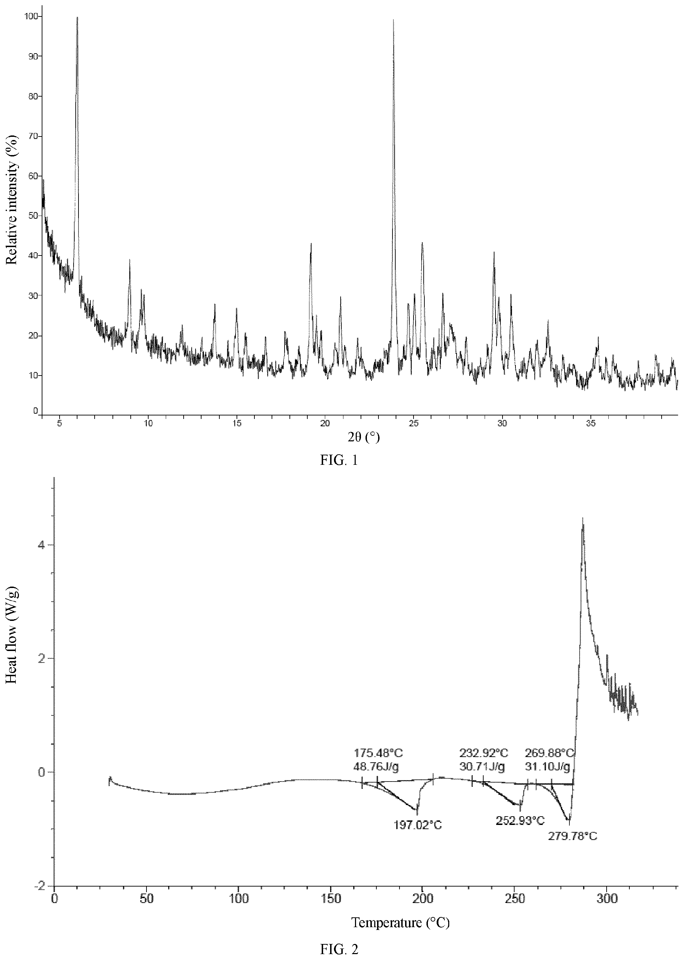

The dried compound (500 mg) was weighed and added into 5 mL of ethanol, and the reaction solution was stirred at 40-50 °C for 24 h. The reaction solution was filtered, and the filter cake was collected and dried in a vacuum drying oven at 40 °C to constant weight, to give crystalline form A of the compound of formula (IIA) (an XRPD pattern of crystalline form A is shown in FIG. 1, a DSC pattern thereof in FIG. 2, and a TGA pattern thereof in FIG. 3). ¹H NMR (400 MHz, deuterated methanol) δ = 9.45 (br s, 1H), 9.04-8.89 (m, 2H), 8.76 (br s, 1H), 8.46 (s, 1H), 8.00 (br d, *J* = 9.0 Hz, 1H), 7.38 (s, 1H), 6.87 (br s, 1H), 4.02 - 3.41 (m, 13H), 3.29 (br d, *J* = 8.3 Hz, 2H), 3.02 - 2.67 (m, 5H), 2.27 (br d, *J* = 9.0 Hz, 2H), 2.13 (s, 3H), 2.06 (d, *J* = 14.4 Hz, 8H).

**Table 5. XRPD diffraction peaks of crystalline form A of the compound of formula (II) in Example 2A**

| **Nos.** | **2θ (°)** | **Relative intensity (%)** | **Nos.** | **2θ (°)** | **Relative intensity (%)** |
|---|---|---|---|---|---|
| 1 | 6.01 | 100 | 18 | 25.05 | 14.2 |
| 2 | 8.95 | 6.6 | 19 | 25.50 | 23.9 |
| 3 | 9.78 | 7.3 | 20 | 26.13 | 8 |
| 4 | 11.93 | 10.3 | 21 | 26.66 | 17.2 |
| 5 | 13.76 | 8.9 | 22 | 27.14 | 26.1 |
| 6 | 15.01 | 7.8 | 23 | 27.95 | 8.1 |
| 7 | 15.50 | 10.1 | 24 | 29.54 | 19.3 |
| 8 | 16.63 | 4.6 | 25 | 29.82 | 26 |
| 9 | 17.87 | 8 | 26 | 30.53 | 23.3 |
| 10 | 19.20 | 19.1 | 27 | 31.60 | 14.1 |
| 11 | 19.75 | 8.1 | 28 | 31.99 | 12.7 |
| 12 | 19.79 | 7.8 | 29 | 32.60 | 14.6 |
| 13 | 20.55 | 7.4 | 30 | 35.42 | 15.2 |
| 14 | 20.88 | 17 | 31 | 35.85 | 6.8 |
| 15 | 22.03 | 9 | 32 | 36.27 | 7.3 |
| 16 | 23.86 | 50.7 | 33 | 38.69 | 10.3 |
| 17 | 24.69 | 15.6 | | | |

The DSC curve of crystalline form A prepared in this example has starting points of endothermic peaks at 175.48 °C, 232.92 °C, and 269.88 °C.

The TGA curve of crystalline form A prepared in this example has a weight loss of 3.439% at 86.3 °C, 8.051% at 160.34 °C, 12.47% at 219.18 °C and 16.33% at 258.50 °C.

### Example 2B: Preparation of Crystalline Form A of the Compound of Formula (IIA)

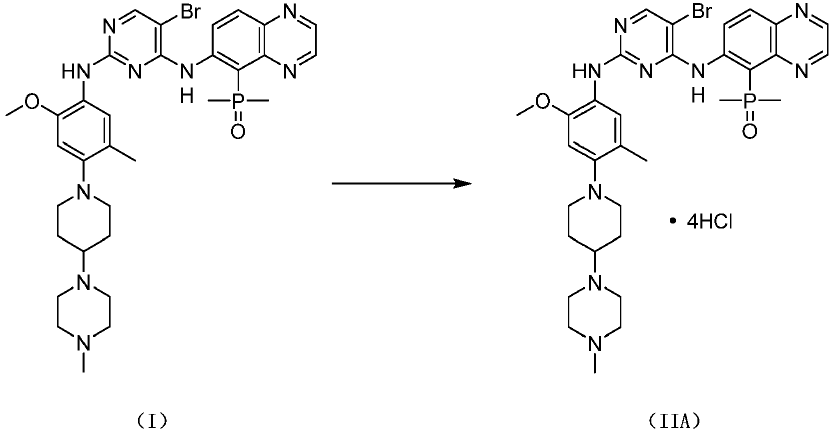

Tetrahydrofuran (12 L) and purified water (0.8 L) were added into a 50 L reaction kettle, and the reaction solution was heated up to 50-60 °C. Then the reaction solution was added with the compound of formula (I) (900 g), stirred until the compound had dissolved, added with activated carbon (80 g), and stirred for 10 min. The reaction solution was filter-pressed to a clean area, the filtrate was cooled to 30-40 °C with stirring for 1 h, further cooled to 10-20 °C with stirring for 6 h and filtered, and the filter cake was dried at 45-55 °C under reduced pressure for 8 h to give a solid (800 g).

Absolute ethanol (12 L) and the prepared solid were added sequentially into a 50 L reaction kettle, and the reaction solution was heated to 75-78 °C. Concentrated hydrochloric acid (36%-38%, 480 mL) diluted with absolute ethanol (4 L) was added dropwise into the reaction kettle. The reaction solution was stirred at 75-78 °C for 2 h, and further stirred at 20-30 °C for 6 h to precipitate a large amount of solid. The solid was filtered, and the filter cake was added with ethanol (5 L) and stirred at 20-30 °C for 0.5 h. The filter cake was collected and dried under reduced pressure at 45-55 °C for 10 h to give crystalline form A of the compound of formula (IIA) (670 g) (an XRPD pattern is shown in FIG. 13).

**Table 6. XRPD diffraction peaks of crystalline form A of the compound of formula (II) in Example 2B**

| **Nos.** | **2θ (°)** | **Relative intensity (%)** | **Nos.** | **2θ (°)** | **Relative intensity (%)** |
|---|---|---|---|---|---|
| 1 | 5.95 | 44.4 | 16 | 21.69 | 12.5 |
| 2 | 8.81 | 11.3 | 17 | 23.32 | 10.3 |
| 3 | 9.51 | 81.4 | 18 | 23.76 | 27.2 |
| 4 | 9.65 | 69.5 | 19 | 24.60 | 11.6 |
| 5 | 12.89 | 19.2 | 20 | 24.95 | 44.0 |
| 6 | 13.61 | 22.0 | 21 | 25.39 | 43.6 |
| 7 | 14.91 | 37.7 | 22 | 26.01 | 11.6 |
| 8 | 15.37 | 12.2 | 23 | 26.27 | 12.8 |
| 9 | 15.78 | 14.1 | 24 | 27.08 | 21.2 |
| 10 | 16.48 | 24.1 | 25 | 29.45 | 14.1 |
| 11 | 17.62 | 15.7 | 26 | 29.67 | 10.6 |
| 12 | 19.04 | 100.0 | 27 | 30.07 | 11.9 |
| 13 | 19.36 | 35.4 | 28 | 31.50 | 14.6 |
| 14 | 19.48 | 16.0 | 29 | 31.88 | 21.4 |
| 15 | 20.42 | 17.6 | | | |

The crystalline forms obtained in Examples 2A and 2B of the present application have the same or similar characteristic peaks in their XRPD patterns according to the analysis of the angle 2θ (°). Therefore, the crystalline forms obtained in Examples 2A and 2B are the same crystalline form.

### Example 3: Preparation of Crystalline Form A of the Compound of Formula (IIA)

Crystalline form A of the compound of formula (IIA) (500 mg) was weighed and added into a 20 mL glass vial and an appropriate amount of ethanol was added to form a suspension. After a magnetic stir bar was added, the suspension was placed on a magnetic heating stirrer (40 °C) for testing (keeping out of the light), stirred at 40 °C for 1 day and then centrifuged, and the residual solid was placed in a vacuum drying oven (30 °C) for drying overnight (10-16 h) to give the crystalline form A of the compound of formula (IIA).

Crystalline form A of the compound of formula (IIA) (500 mg) was weighed and added into a 20 mL glass vial and an appropriate amount of isopropanol was added to form a suspension. After a magnetic stir bar was added, the suspension was placed on a magnetic heating stirrer (40 °C) for testing (keeping out of the light), stirred at 40 °C for 1 day and then centrifuged, and the residual solid was placed in a vacuum drying oven (30 °C) for drying overnight (10-16 h) to give the crystalline form A of the compound of formula (IIA).

Crystalline form A of the compound of formula (IIA) (500 mg) was weighed and added into a 20 mL glass vial and an appropriate amount of tetrahydrofuran was added to form a suspension. After a magnetic stir bar was added, the suspension was placed on a magnetic heating stirrer (40 °C) for testing (keeping out of the light), stirred at 40 °C for 1 day and then centrifuged, and the residual solid was placed in a vacuum drying oven (30 °C) for drying overnight (10-16 h) to give the crystalline form A of the compound of formula (IIA).

Crystalline form A of the compound of formula (IIA) (500 mg) was weighed and added into a 20 mL glass vial and an appropriate amount of acetonitrile was added to form a suspension. After a magnetic stir bar was added, the suspension was placed on a magnetic heating stirrer (40 °C) for testing (keeping out of the light), stirred at 40 °C for 1 day and then centrifuged, and the residual solid was placed in a vacuum drying oven (30 °C) for drying overnight (10-16 h) to give the crystalline form A of the compound of formula (IIA).

Crystalline form A of the compound of formula (IIA) (500 mg) was weighed and added into a 20 mL glass vial and an appropriate amount of *n*-heptane was added to form a suspension. After a magnetic stir bar was added, the suspension was placed on a magnetic heating stirrer (40 °C) for testing (keeping out of the light), stirred at 40 °C for 1 day and then centrifuged, and the residual solid was placed in a vacuum drying oven (30 °C) for drying overnight (10-16 h) to give the crystalline form A of the compound of formula (IIA).

Crystalline form A of the compound of formula (IIA) (500 mg) was weighed and added into a 20 mL glass vial and an appropriate amount of ethyl acetate was added to form a suspension. After a magnetic stir bar was added, the suspension was placed on a magnetic heating stirrer (40 °C) for testing (keeping out of the light), stirred at 40 °C for 1 day and then centrifuged, and the residual solid was placed in a vacuum drying oven (30 °C) for drying overnight (10-16 h) to give the crystalline form A of the compound of formula (IIA).

Crystalline form A of the compound of formula (IIA) (500 mg) was weighed and added into a 20 mL glass vial and an appropriate amount of acetone was added to form a suspension. After a magnetic stir bar was added, the suspension was placed on a magnetic heating stirrer (40 °C) for testing (keeping out of the light), stirred at 40 °C for 1 day and then centrifuged, and the residual solid was placed in a vacuum drying oven (30 °C) for drying overnight (10-16 h) to give the crystalline form A of the compound of formula (IIA).

### Example 4: Preparation of Crystalline Form B of the Compound of Formula (IIA)

Crystalline form A of the compound of formula (IIA) (500 mg) was weighed and added into a 20 mL glass vial and an appropriate amount of methanol was added to form a suspension. After a magnetic stir bar was added, the suspension was placed on a magnetic heating stirrer (40 °C) for testing (keeping out of the light), stirred at 40 °C for 1 day and then centrifuged, and the residual solid was placed in a vacuum drying oven (30 °C) for drying overnight (10-16 h) to give the crystalline form B of the compound of formula (IIA).

### Example 5: Preparation of Crystalline Form C of the Compound of Formula (IIA)

Crystalline form A of the compound of formula (IIA) (500 mg) was weighed and added into a 100 mL glass vial and an appropriate amount of a mixed solvent of methanol and water (methanol:water = 10:1 (v/v)) was added to form a suspension. After a magnetic stir bar was added, the suspension was placed on a magnetic heating stirrer (70-80 °C) for testing (keeping out of the light), stirred for 2 h, then cooled to 20-30 °C with stirring overnight, and then centrifuged, and the residual solid was placed in a vacuum drying oven (40 °C) for drying overnight (10-16 h) to give the crystalline form C of the compound of formula (IIA).

Crystalline form A of the compound of formula (IIA) (500 mg) was weighed and added into a 100 mL glass vial and an appropriate amount of a mixed solvent of ethanol and water (ethanol:water = 2:1 (v/v)) was added to form a suspension. After a magnetic stir bar was added, the suspension was placed on a magnetic heating stirrer (70-80 °C) for testing (keeping out of the light), stirred for 2 h, then cooled to 20-30 °C with stirring overnight, and then centrifuged, and the residual solid was placed in a vacuum drying oven (40 °C) for drying overnight (10-16 h) to give the crystalline form C of the compound of formula (IIA).

Crystalline form A of the compound of formula (IIA) (500 mg) was weighed and added into a 100 mL glass vial and an appropriate amount of a mixed solvent of isopropanol and H₂O (isopropanol:H₂O = 5:1 (v/v)) was added to form a suspension. After a magnetic stir bar was added, the suspension was placed on a magnetic heating stirrer (70-80 °C) for testing (keeping out of the light), stirred for 2 h, then cooled to 20-30 °C with stirring overnight, and then centrifuged, and the residual solid was placed in a vacuum drying oven (40 °C) for drying overnight (10-16 h) to give the crystalline form C of the compound of formula (IIA).

Crystalline form A of the compound of formula (IIA) (500 mg) was weighed and added into a 100 mL glass vial and an appropriate amount of a mixed solvent of isopropanol and water (isopropanol:water = 3:1 (v/v)) was added to form a suspension. After a magnetic stir bar was added, the suspension was placed on a magnetic heating stirrer (70-80 °C) for testing (keeping out of the light), stirred for 2 h, then cooled to 20-30 °C with stirring overnight, and then centrifuged, and the residual solid was placed in a vacuum drying oven (40 °C) for drying overnight (10-16 h) to give the crystalline form C of the compound of formula (IIA).

### Example 6: Preparation of Crystalline Form D of the Compound of Formula (IIA)

Crystalline form A of the compound of formula (IIA) (500 mg) was weighed and added into a 100 mL glass vial and an appropriate amount of a mixed solvent of ethanol and water (ethanol:water = 5:1 (v/v)) was added to form a suspension. After a magnetic stir bar was added, the suspension was placed on a magnetic heating stirrer (70-80 °C) for testing (keeping out of the light), stirred for 2 h, then cooled to 20-30 °C with stirring overnight, and then centrifuged, and the residual solid was placed in a vacuum drying oven (40 °C) for drying overnight (10-16 h) to give the crystalline form D of the compound of formula (IIA).

Crystalline form A of the compound of formula (IIA) (500 mg) was weighed and added into a 100 mL glass vial and an appropriate amount of a mixed solvent of acetone and water (acetone:water = 5:1 (v/v)) was added to form a suspension. After a magnetic stir bar was added, the suspension was placed on a magnetic heating stirrer (70-80 °C) for testing (keeping out of the light), stirred for 2 h, then cooled to 20-30 °C with stirring overnight, and then centrifuged, and the residual solid was placed in a vacuum drying oven (40 °C) for drying overnight (10-16 h) to give the crystalline form D of the compound of formula (IIA).

### Example 7: Ion Chromatography Detection of Chloride Ions

Two parts of crystalline form A of the compound of formula (IIA) (about 20 mg) were precisely weighed into two 20 mL volumetric flasks, and dissolved and diluted with a solvent to volume; two parts of the above solutions (1 mL) were precisely transfered into two 20 mL volumetric flasks, respectively, and dissolved and diluted with a solvent to volume, and the solutions were labeled, respectively. The content of chloride ions was then detected by ion chromatograph, and the detection results are shown in Table 7.

**Table 7**

| **Sample** | **Weight (mg)** | **Chloride ion content (%)** | **Average chloride ion content (%)** |
|---|---|---|---|
| Crystalline form A of the compound of formula (IIA) | 20.77 | 16.78 | 16.8 |
| | 20.33 | 16.75 | |

It is concluded that after the content of the chloride ions is detected by a chloride ion detector, the number of the chlorine atoms in crystalline form A of the compound (IIA) is about 4 by formula calculations.

### Test example 1: study on in vivo efficacy of the compound of formula (I) (1)

### Experimental method:

*In vivo* drug efficacy experiment was performed on xenograft (CDX) BALB/c nude mice subcutaneously implanted with Ba/F₃ (Δ19 del/T790M/C797S) source. BALB/c nude mice (female, 6-8 weeks, weighing about 18-22 g) were bred in SPF-rated environments, and each cage was ventilated individually (5 mice per cage). All animals were free to obtain a standard certified commercial laboratory diet. 48 mice in total were purchased from Beijing Vital River Laboratory Animal Technology Co., Ltd for the study. Each mouse was implanted with cells subcutaneously in the right flank for tumor growth. The experiment was started when the mean tumor volume reached about 80-120 mm³. The test compound was administered orally daily with the compound of formula (I) (5 mg/kg, 15 mg/kg, 45 mg/kg, respectively) administered for 13 consecutive days, and the data are shown in Table 5. Tumor volume was measured twice weekly with a two-dimensional caliper, and the volume was measured in mm³ and calculated according to the following formula: V = 0.5a × b², where a and b are the long and short diameters, respectively, of the tumor. Anti-tumor efficacy was determined by dividing the mean tumor increase volume of compound-treated animals by the mean tumor increase volume of untreated animals. The inhibition of tumor growth by the test compound *in vivo* was evaluated by the tumor volume inhibition value (TGI), wherein the TGI of the group administered with the compound of formula (I) (15 mg/kg) was 74.9%, and the TGI of the group administered with the compound of formula (I) (45 mg/kg) was 101.69%.

On day 14 after administration to the groups for the efficacy experiment, before the last administration and 2 h after the last administration, the plasma of the mice was collected by blood collection from the submandibular vein, and the plasma of the mice was collected at 1 h, 4 h, 8 h, and 24 h after the administration. About 100 µL of blood was collected each time, placed in an anticoagulation tube, and centrifuged at 8000 rpm for 7 min to collect blood plasma, and the blood plasma was preserved at -80 °C. Tissues of mouse lung and tumor were collected at the same time 2 h after the administration and stored at -80 °C. Wherein the tumors were divided into two parts (the tumor weight for PD analysis did not exceed 100 mg) for detection and data analysis. The experimental results are shown in Tables 8 and 9.

**Table 8**

| Test compound | Dosage | Tumor volume (mm³) | | | | | | |
|---|---|---|---|---|---|---|---|---|
| | | Day 0 | Day 2 | Day 5 | Day 8 | Day 10 | Day 12 | Day 13 |
| Blank Control | N/A | 85 | 143 | 315 | 582 | 765 | 929 | 1048 |
| Compound of formula (I) | 5 mg/kg/day | 84 | 108 | 212 | 395 | 505 | 748 | 881 |
| | 15 mg/kg/day | 84 | 76 | 107 | 126 | 176 | 292 | 326 |
| | 45 mg/kg/day | 84 | 56 | 32 | 35 | 42 | 73 | 68 |

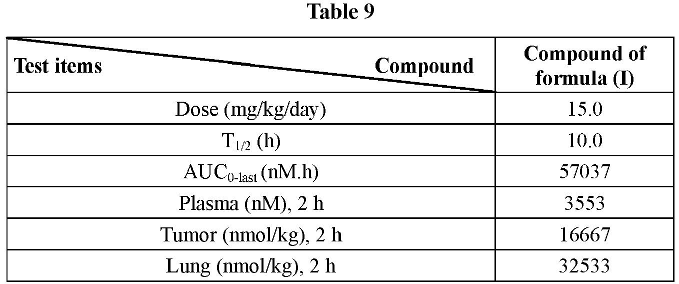

### Test example 2: study on in vivo efficacy of the compound of formula (I) (2)

### Experimental method:

1. Cell culture: lung cancer PC-9 cells were cultured in an RPMI 1640 medium containing 10% fetal bovine serum, 100 U/mL penicillin and 100 µg/mL streptomycin through *in vitro* monolayer culture in an incubator at 37 °C/5% CO₂. The cells were digested with trypsin-EDTA twice a week for passaging as per conventional practice. At a cell saturation of 80%-90% and a required number, the cells were collected and counted. The cell density was 5 × 10⁶ cells.
2. Cell inoculation: 0.2 mL (containing 5×10⁶) of a suspension of PC-9 cells (PBS:Matrigel = 1:1) was subcutaneously inoculated in the right back of each mouse, and 64 mice were inoculated in total. On day 7 after inoculation, when mean tumor volume was measured to be 169 mm³, grouping administration was performed using a randomized stratified grouping method based on tumor volume and mice body weight. PBS is phosphate buffered saline, and Matrigel is matrigel.
3. Administration: the dosage was 50 mg/kg for 0-9 days and 25 mg/kg for 10-21 days; the drug was orally administrated once a day for 3 weeks.

### Tumor measurement and experimental indices

Tumor diameters were measured twice weekly using a vernier caliper. The tumor volume was calculated using the following formula: V = 0.5a × b², where a and b represent the long diameter and short diameter of the tumor respectively.

The efficacy of compounds against tumor was evaluated by TGI (%).

Relative tumor volume (RTV) was calculated based on the results of tumor measurement. The formula is RTV = Vₜ/V₀, where V₀ represents the tumor volume measured at the time point of grouping and administration (i.e., D₀), and Vₜ represents the tumor volume at a certain measurement for the corresponding mouse, the data of T_{RTV} and C_{RTV} should be measured on the same day.

TGI (%) reflects the tumor growth inhibition rate. TGI (%) = [(1 - (average tumor volume at the end of administration in a treatment group - average tumor volume at the start of administration of the treatment group)) / (average tumor volume at the end of treatment of the solvent control group - average tumor volume at the start of treatment of the solvent control group)] × 100%.

Tumor weights will be measured at the end of the experiment and the TGI (%) was calculated.

**Experimental results** are shown in Table 10. The TGI of the compound of formula (I) was 100% at day 23.

**Table 10**

| Test compound | Dose | Tumor volume (mm³) | | | | | | | |
|---|---|---|---|---|---|---|---|---|---|
| | | Day 0 | Day 2 | Day 6 | Day 9 | Day 13 | Day 16 | Day 20 | Day 23 |
| Blank Control | / | 186 | 257 | 285 | 326 | 482 | 527 | 637 | 921 |
| Compound of formula (I) | 50 mg/kg (0-9 days) | 185 | 198 | 92 | 75 | 40 | 45 | 76 | 111 |
| | 25 mg/kg (10-21 days) | | | | | | | | |

### Test example 3: stability test of crystalline form A of the compound of formula (II) in Example 2A

### Experimental instruments:

Illumination test chamber, model: SHH-100GD-2, conditions: 5000±500 lux (visible light) and 90 mw/cm² (ultraviolet).

Electric heating blast drying oven, model: GZX-9140MBE condition: 60 °C.

Constant temperature and humidity chamber, model: SHH-250SD, condition: 25 °C/75% RH.

Constant temperature and humidity chamber, model: LDS-800Y, condition: 40 °C/75% RH and 25 °C/60% RH. Experimental method:
Influencing factor experimental conditions (high temperature of 60 °C, high humidity of 75% RH, illumination): an appropriate amount of crystalline form A of the compound of formula (IIA) was placed in a dry and clean open weighing bottle, then the bottle was placed into a dryer under different conditions, and then placed into a corresponding thermostat for observation. The bottle was taken out after being placed for 5 days, 10 days and 30 days, and related substances and contents thereof were measured.

Accelerated and long-term experimental conditions (40 °C/75% RH, 25 °C/60% RH): an appropriate amount of crystalline form A of the compound of formula (IIA) was filled into a double-layer LDPE bags, each layer of LDPE bag was sealed by a binding buckle, then put into an aluminum foil bag for heat sealing, and then put into a corresponding thermostat to observe acceleration and long-term stability.

### Experimental results:

The content changes of crystalline form A of the compound of formula (IIA) under the conditions of illumination, high temperature and high humidity are shown in Table 11.

**Table 11. Experiments of influencing factors**

| **Items** | **Initial value** | **Light stability experiment** | | **High temperature (60 °C)** | | | **High humidity (25 °C/75% RH)** |
|---|---|---|---|---|---|---|---|
| | | **Day 5** | **Day 10** | **Day 5** | **Day 10** | **Day 30** | **Day 5** |
| **Total impurities** | 1.63% | 1.99% | 2.21% | 1.59% | 1.63% | 1.71% | 1.41% |
| **Content** | 97.3% | 97.1% | 95.1% | 99.1% | 101.6% | 99.4% | 99.6% |

| | | | | | | | |
|---|---|---|---|---|---|---|---|
| Note: N/A indicates no detection. | | | | | | | |

The content changes of crystalline form A of the compound of formula (IIA) under accelerated and long-term conditions are shown in Tables 12 and 13.

**Table 12. Results of accelerated experiments (40 °C±2 °C/75% RH±5% RH)**

| **Detection items** | **Observation time** | | | | |
|---|---|---|---|---|---|
| | **0 day** | **1 month** | **2 months** | **3 months** | **6 months** |
| **Total impurities** | 1.63% | 1.71% | 1.50% | 1.56% | 1.61% |
| **Content** | 97.3% | 99.1% | 98.1% | 98.6% | 97.9% |

**Table 13. Results of long-time experiments (25 °C±2 °C/60% RH±5% RH)**

| **Detection items** | **Observation time** | | | | |
|---|---|---|---|---|---|
| | **0 day** | **3 months** | **6 months** | **9 months** | **12 months** |
| **Total impurities** | 1.63% | 1.61% | 1.73% | 1.83% | 1.69% |
| **Content** | 97.3% | 99% | 98.2% | 99.4% | 97.7% |

### Test example 4: stability test of crystalline form A of the compound of formula (II) in Example 2B

The conditions and the method for the raw material stability test are mainly based on related requirements such as "Guidelines for the Stability Test of APIs and Preparations" (General Principles 9001 of the Four Parts of the Chinese Pharmacopoeia, 2015 Edition) and technical guidelines for stability study on chemical drugs (raw materials and preparations) issued by the National Medical Products Administration in February, 2015 (revised), and are specifically shown in Table 14.

**Table 14**

| **Experimental condition** | | **Observation time points** | **Sample amount** |
|---|---|---|---|
| Accelerated test | 40 °C±2 °C, 75% | Month 1, month 2, month 3 and month 6 | 1.0 g/bag, 5 bags |
| | RH±5% RH | | 0.7 g/bag, 5 bags |

**Experimental results are shown in Table 15.**

**Table 15**

| **Observation items** | **Limit requirements** | **Time (month)** | | | | |
|---|---|---|---|---|---|---|
| | | **0** | **1** | **2** | **3** | **6** |
| Sum of impurities | ≤3.0% | 1.18 | 1.10 | 1.18 | 1.30 | 1.18 |
| Water (%) | ≤7.0% | 3.0 | 3.0 | 3.5 | 3.2 | 2.8 |

## Claims

1. A compound of formula (II), 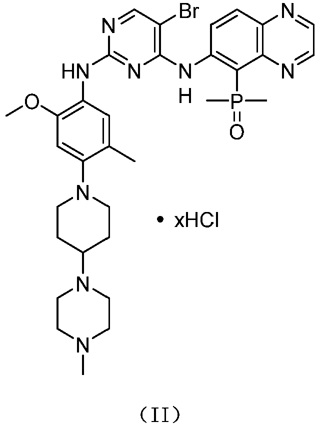 wherein x is selected from 3.0-5.0; preferably 3.2-4.8, more preferably 3.5-4.5, further preferably 3.8-4.2, and further more preferably 4.0.

2. The compound of formula (II) according to claim 1, wherein the compound is in a solid form, optionally the solid form is selected from the group consisting of amorphous form and crystalline form.

3. Crystalline form A of the compound of formula (II) of claim 1 or 2, wherein the crystalline form A has diffraction peaks at the following 2θ of 5.95°, 9.51° and 19.04°; typically has diffraction peaks at the following 2θ of 5.95°, 9.51°, 14.91°, 19.04°, 24.95° and 25.39°; more typically has diffraction peaks at the following 2θ of 5.95°, 9.51°, 14.91°, 16.48°, 19.04°, 23.76°, 24.95°, 25.39° and 27.08°; further typically has diffraction peaks at the following 2θ of 5.95°, 9.51°, 9.65°, 14.91°, 16.48°, 19.04°, 19.36°, 23.76°, 24.95°, 25.39° and 27.08°; and further more typically has diffraction peaks at the following 2θ of 5.95°, 9.51°, 9.65°, 13.61°, 14.91°, 16.48°, 19.04°, 19.36°, 23.76°, 24.95°, 25.39°, 27.08° and 31.88°, in the X-ray powder diffraction pattern thereof.

4. The crystalline form A according to claim 3, wherein the X-ray powder diffraction pattern thereof is shown in FIG. 13.

5. A method for preparing the crystalline form A of the compound of formula (II) of claim 3 or 4, comprising:
(a) adding the compound of formula (I) in a solvent, and heating the resulting solution up to 70-80 °C;
(b) dissolving hydrochloric acid in a solvent, slowly adding with the solution prepared in step (a), and stirring at 70-80 °C for 1-2 h; and
(c) cooling the solution to 20-30 °C, stirring for 12 h, filtering under reduced pressure and N₂ atmosphere protection, and drying;
wherein the solvent is ethanol.

6. Crystalline form B of the compound of formula (II) of claim 1 or 2, wherein the crystalline form B has characteristic diffraction peaks at the following 2θ of 6.39°, 12.39° and 14.18°; typically has diffraction peaks at the following 2θ of 6.39°, 12.39°, 13.03°, 14.18°, 19.03° and 26.76°; more typically has diffraction peaks at the following 2θ of 6.39°, 12.39°, 13.03°, 14.18°, 18.52°, 19.03°, 26.09° and 26.76°; and further typically has diffraction peaks at the following 2θ of 6.39°, 7.05°, 12.39°, 13.03°, 14.18°, 16.64°, 18.52°, 19.03°, 26.09° and 26.76°, in the X-ray powder diffraction pattern thereof.

7. The crystalline form B according to claim 6, wherein the X-ray powder diffraction pattern thereof is shown in FIG. 4.

8. A method for preparing the crystalline form B of the compound of formula (II) of claim 6 or 7, comprising:
(a) adding the compound of formula (II) to a solvent, to form a suspension;
(b) stirring the suspension at 35-45 °C for 24 h; and
(c) drying at 30-40 °C for 8-16 h after centrifugation;
wherein the solvent is methanol.

9. Crystalline form C of the compound of formula (II) of claim 1 or 2, wherein the crystalline form C has characteristic diffraction peaks at the following 2θ of 6.78°, 10.33° and 14.04°; typically has diffraction peaks at the following 2θ of 6.78°, 10.33°, 14.04°, 18.66°, 20.37° and 25.66°; more typically has diffraction peaks at the following 2θ of 6.78°, 10.33°, 14.04°, 18.66°, 20.37°, 25.09°, 25.66° and 26.62°; and further typically has diffraction peaks at the following 2θ of 6.78°, 10.33°, 13.65°, 14.04°, 18.66°, 20.37°, 21.04°, 25.09°, 25.66° and 26.62°, in the X-ray powder diffraction pattern thereof.

10. The crystalline form C according to claim 9, wherein the X-ray powder diffraction pattern thereof is shown in FIG. 7.

11. A method for preparing the crystalline form C of the compound of formula (II) of claim 9 or 10, comprising:
(a) adding the compound of formula (II) to a solvent, to form a suspension;
(b) stirring the suspension at 70-80 °C for 1-2 h, the suspension becoming clear during stirring;
(b) cooling the suspension to 20-30 °C with stirring for 12 h, a solid being precipitated during cooling; and
(c) drying the solid at 30-40 °C for 8-16 h after centrifugation;
wherein the solvent is selected from a mixed solvent of methanol and water, and the volume ratio of methanol to water in the mixed solvent is 10:1.

12. Crystalline form D of the compound of formula (II) of claim 1 or 2, wherein the crystalline form D has characteristic diffraction peaks at the following 2θ of 7.08°, 10.53° and 12.10°; typically has diffraction peaks at the following 2θ of 7.08°, 9.11°, 10.53°, 12.10°, 13.78° and 17.51°; more typically has diffraction peaks at the following 2θ of 7.08°, 9.11°, 10.53°, 12.10°, 13.78°, 17.51°, 20.21° and 24.18°; and further typically has diffraction peaks at the following 2θ of 7.08°, 7.92°, 9.11°, 10.53°, 12.10°, 13.78°, 16.15°, 17.51°, 20.21°, 24.18° and 26.11°, in the X-ray powder diffraction pattern thereof.

13. The crystalline form D according to claim 12, wherein the X-ray powder diffraction pattern thereof is shown in FIG. 10.

14. A method for preparing the crystalline form D of the compound of formula (II) of claim 12 or 13, comprising:
(a) adding the compound of formula (II) to a solvent, to form a suspension;
(b) stirring the suspension at 70-80 °C for 1-2 h, the suspension becoming clear during stirring;
(b) cooling the suspension to 20-30 °C with stirring for 12 h, a solid being precipitated during cooling; and
(c) drying the solid at 30-40 °C for 8-16 h after centrifugation;
wherein the solvent is selected from a mixed solvent of ethanol and water, and the volume ratio of ethanol to water in the mixed solvent is 5:1.

15. A crystalline form composition, comprising the crystalline form A of claim 3 or 4, the crystalline form B of claim 6 or 7, the crystalline form C of claim 9 or 10, or the crystalline form D of claim 12 or 13, wherein each of the crystalline forms accounts for 50wt% or more, preferably 80wt% or more, more preferably 90wt% or more, and most preferably 95wt% or more of the crystalline form composition.

16. A pharmaceutical composition, comprising a therapeutically effective amount of the compound of formula (II) of claim 1 or 2, the crystalline form A of claim 3 or 4, the crystalline form B of claim 6 or 7, the crystalline form C of claim 9 or 10, or the crystalline form D of claim 12 or 13, or the crystalline form composition of claim 15, and optionally, a pharmaceutically acceptable carrier, excipient and/or vehicle

17. Use of the compound of formula (II) of claim 1 or 2, the crystalline form A of claim 3 or 4, the crystalline form B of claim 6 or 7, the crystalline form C of claim 9 or 10, or the crystalline form D of claim 12 or 13, the crystalline form composition of claim 15, or the pharmaceutical composition of claim 16 for the preparation of a medicament for treating an EGFR-related disease.
